# EUROPEAN PATENT APPLICATION

(11) **EP 2 067 771 A1**
(43) Date of publication of application: **10.06.2009**
(21) Application number: 07122188.1
(22) Date of filing: 03.12.2007
(51) Int. Cl.: C07D 207/12, C07D 491/056, A61K 31/40, A61P 35/00

(54) **Derivatives of Dihydroxypyrrolidine as Anti-Cancer Compounds**

(71) Applicant: EPFL Ecole Polytechnique Fédérale de Lausanne, 1015 Lausanne (CH)
(72) Inventor: The designation of the inventor has not yet been filed
(74) Representative: Schneiter, Sorin

(57) **Abstract**

The present invention relates to derivatives of dihydroxypyrrolidine useful in the treatment of cancer. The invention further relates to a process for making the compounds. The compounds are inhibitors of α mannosidase, and possibly, also inhibit nicotinamide phosphoribosyl transferase.

## Description

### Field of the Invention

The present invention relates to novel compounds, the use of these compounds as medicaments, in particular in the treatment of cancer, as well as a method for producing such compounds.

### Background of the invention and Problems to be Solved

The specific inhibition of α-mannosidases has already been proposed as an anti-cancer strategy, in particular because catabolic and processing glycosidases were shown to be involved in the transformation of normal cells to cancer cells.

H. Fiaux et al. (J. Med. Chem. 2005, 48, 4237-46) disclose functionalised pyrrolidines that inhibit α-mannosidase and growth of human glioblastoma and melanoma cells. However, several of the dihydroxypyrrodlidine derivatives disclosed in this paper actually had high inhibitory effects on α-mannosidase but only little or no anti-cancer effect. Interestingly, swainsonine, an α-mannosidase inhibitor of which anti-tumoral properties were reported previously, had only little inhibitory effect on glioblastoma cell growth.

S. Favre et al. (Heterocylces, Vol. 69, 2006) report 2-benzylamino-3,4-dihydroxypyrrolidines bearing aromatic and aliphatic amido side chains as specific inhibitors of α-mannosidase and of the growth of human glioblastoma cells. While many of the compounds disclosed in this reference show inhibititory effects of α-mannosidase, only one specimen, (N-[(2*R*)-2-({[(2*R*,3*R*,4*S*)-3,4-dihydroxypyrrolidin-2-yl]methyl}amino)-2-phenylethyl]-3-bromobenz-amide) showed convincing inhibition of human glioblastoma cells.

In view of the prior art, it is an objective of the present invention to provide new derivatives of dihydroxypyrrolidines that are useful in the treatment of cancer.

It is a further objective to provide compounds that are capable of attacking at several targets, not only the α-mannosidases. It is a particular objective underlying the present invention to provide derivatives of dihydroxypyrrolidines that inhibit, besides α-mannosidases, also nicotinamide phosphoribosyltransferase.

It is another objective of the present invention to provide new compounds that are suitable to specifically inhibit tumor cells while not or only to a lesser extent affecting healthy cells.

A further objective of the present invention is to provide compounds useful in the treatment of cancer, wherein said compounds show improved internalization by the tumor cells if compared to compounds of the prior art.

It is another objective of the present invention to provide a new anticancer strategy able to overcome resistance to conventional chemotherapeutic agents, especially for the treatment of human glioblastome and metastatic melanoma, for which only very few therapeutic options exist by now.

It is a further objective of the present invention to provide a method for producing new derivatives of dihydroxypyrrolidines. Such a method preferably includes as few steps as possible and preferably obtains high yields in most of the steps. Preferably, intermediate and final products are obtained with high enantiomeric excess.

### Summary of the invention

Remarkably, the present inventors provide new derivatives of dihydroxypyrrolidines which differ from compounds of the prior art. Surprisingly, *in vitro* tests revealed excellent anti-cancerous properties of such compounds. Without wishing to be bound by theory it is speculated that these compounds are inhibitors of α-mannosidase. According to a further possibility, the compound of the present invention may inhibit phosphoribosyl transferases, simultaneously or alternatively to α-mannosidase.

Accordingly, the present invention provides a compound of formula (I): 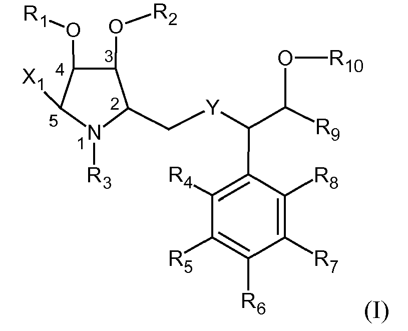 wherein:
X₁ is selected from H, -CH₂-O-R, and -O-R;
Y is selected from-NR₁₁-, -O-, CO, CH(OH), CH(NH₂), -CH₂-, -S-, -SO-, -SO₂-;
R, R₁, R₂, R₃, R₄, R₅, R₆, R₇, R₈ R₉, R₁₀ and, if applicable R₁₁, independently of each other, from H, C1-C26 alkyl, C1-C26 acyl, C2-C26 alkenyl, C2-C26 alkynyl, C6-C26 aryl,
wherein said alkyl, acyl, alkenyl, alkynyl, and aryl, may be further substituted and may comprise one or more heteroatoms;
wherein, furthermore, R₁, R₂ may form an acetal of formula (II) 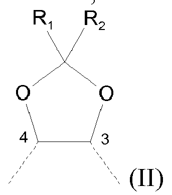 wherein, in said acetal, R₁ and R₂ are as defined above;
wherein at least one of R₄-R₈ is different from hydrogen;
wherein any carbon-bound H in any of the residues R₁-R₁₁ may be replaced by halogen;
wherein -R₉ may also represent a carbonyl group (=O);
wherein the compound of formula (I) may be provided in the form of a pharmaceutically acceptable salt.

In a further aspect, the present invention provides a pharmaceutical composition comprising the compound and a method for preparing compound of the invention.

The invention also relates to a method of treating cancer, comprising the step of administering, to an individual in need thereof, an effective amount of the compounds of the present invention.

Further aspects and preferred embodiments of the present invention are defined herein below and in the appended claims.

### Brief Description of the Figures

**Figure 1** illustrates the synthesis of protected [1,1'-biphenyl]-D-glycine **(36),** an intermediate compound, from commercially available 4-hydroxy-D-phenylglycine **(32).**
**Figure 2** shows the synthesis of the biphenyl ester **(38)** from intermediate **(36)** shown in Figure 1.
**Figure 3** shows the synthesis of a compound of the present invention, the biphenyl **(40),** from the ester **(38)** shown in Figure 2.
**Figure 4** shows the synthesis of thienyl **(47)** and (4'-trifluoromethyl)biphenyl **(48),** further compounds according to the present invention, from intermediate **(35)** shown in Figure 1.
**Figure 5** shows the preparation of allyl, benzyl and methyl ethers (compounds **49, 50** and **51**) of protected 4-hydroxy-D-phenylglycine **(32)** and **(34)** (Figure 1).
**Figure 6** shows the synthesis of enantiomerically pure 2-(*R*)-(4-bromophenyl)glycinol ((R-)-13), an intermediate in the preparation of compound **(11)** according to the present invention.
**Figure 7** shows compounds according to the present invention, their inihibitory activity (IC₅₀) towards α-mannosidase from jack bean and their selectivity (Kᵢ) with respect to other glycosidases.
**Figure 8** shows the viability of U87 (glioblastoma) tumor cell lines following exposure to different concentrations of the compounds of the present invention. CB183, CB161 and CB181 refer to compounds **48, 40** and **52** in Figure 7, respectively.

### Detailed Description of the Preferred Embodiments

The present invention provides compounds according to the structure of formula (I) as medicaments, in particular in the treatment of cancer.

In the compounds of formula (II), any one of R, R₁, R₂, R₃, R₄, R₅, R₆, R₇, R₈ R₉, R₁₀ and, if applicable R₁₁, (hereinafter: R-R₁₁) may independently of the substituents be selected from H, C1-C26 alkyl, C1-C26 acyl, C2-C26 alkenyl, C2-C26 alkynyl, C6-C26 aryl, wherein said alkyl, acyl, alkenyl, alkynyl, and aryl, may be further substituted and may comprise one or more heteroatoms.

For the purpose of the present specification, the term "comprising" refers to "including, amongst other". It is not intended to be construed as "consists only of".

Preferred examples of substituents of R₁ and R₂ are those that form an ester, such as, for example:
(a) substituents of formula -CO-R₁₅, wherein R₁₅ stands for an C1-C6 alkyl, which may be substituted by halogen and/or -OH. Specific examples are -COCH₃; -COCH₂F, -COCH₂Cl, -CO-CH₂(OH), -COCH₂CH₃.
(b) substituents of formula -CO-CO-R₁₆ wherein R₁₆ is selected from H or as R₁₅ as defined above. Specific examples are: -CO-CO-H, -COCOMe, -COCOEt, -COCOCH₂CH₂CH₃.
(c) substituents of formula -CO-R₁₇, wherein R₁₇ may be selected from Ph, and wherein said Ph may be substituted by C1-C5 alkyl as defined below, halogen and/or -OH, preferably at carbon 4 of the phenyl residue. Specific examples include: -CO-Ph, -CO-C₆H₅Br.

R₁ and R₂ in formula (I) may form together an acetal of formula (II): 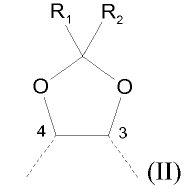 wherein, in said acetal, R₁ and R₂ are as defined above.

Preferred examples of substituents of R₁ and R₂ when forming an acetal of formula (II) in which R₁=R₂= H, C1-C10 alkyl, wherein one or more hydrogens may be substituted by halogen. Specific examples are -CH₃, -CH₂CH₃, etc. In other examples, R₁ and R₂ in formula (II) are different, for example R₁=H and R₂ is selected from C1-C10 alkyl or Ph, wherein one or more hydrogen may be substituted by halogen. Specific examples of formula (II) are: R₁=H, R₂ = Ph, Me, Et, etc.

In the compound of formula (I) above, X may be selected from H, -CH-O-R, or -O-R as defined above.

According to a preferred embodiment, X is selected from -H, -OH, -CH₂OH, -O-R and -CH₂-O-R, with R being a C1-C10 alkyl, acyl, alkenyl, alkynyl, aryl, as defined below, but preferably an alkyl.

In the compound of formula (I), Y is selected from -NR₁₁-, -O-, CO, CH(OH), CH(NH₂), -CH₂-, -S-, -SO-, -SO₂-. Preferably it is -NR₁₁.

In the compound of formula (I), R₃, and, if applicable, R₁₁, are selected, independently of each other, as indicated above. According to a preferred embodiment, R₃, and, if applicable, R₁₁, are selected from H and C1-C10 alkyl as defined below. One or more hydrogen atoms of these alkyls, for example, may be substituted with halogen.

R₃, and, if applicable, R₁₁, may also be selected from acyls as indicated above. Preferred examples are indicated with reference to substituents -CO-R₁₅ and -CO-R₁₇ under (a) and (c) above, respectively. R₃ and R₁₁ may also be selected from the specific examples indicated under (a) and (c) above. Other examples of -CO-R₁₇ include: -CO-C₆H₄-CF₄-4 (at position 4 of the phenyl) and -CO-C₆H₃(CF₃)₂-2,5 (with the CF₃ substituent located at positions 2 and 5 of the phenyl).

Preferably, R₃, and, if applicable, R₁₁, are H.

R₉ is as defined above. Preferably, it is selected from H, C1-C15 alkyl as defined below, and -S-R₁₂-, wherein R₁₂ is a C1-C15 alkyl.

In the compound of formula (I), R₁₀ is preferably selected from the indicated substituents as defined above. Preferably, R₁₀ is selected from C1-C26 alkyls including branched alkyls.

R₁₀ may further preferably be selected from an C1-C26 alkenyl, wherein the alkenyl may comprise 1, 2 or more double bonds. Configuration may be E and/or Z. Preferably, if two or more double bond are present, they are separated by at least one single bond.

R₁₀ may further preferably be selected from substituents having the following formulae:
- (CH₂CH₂-O)_{N}-R₂₀, wherein N is an integer selected from 1-8 and R₂₀ is an alkyl as defined below, preferably a C1-C5 alkyl as defined below.

R₁₀ may further preferably be selected from substituents having the following formulae:
- (CH₂)_{N}-O-(CH₂)_{M}-R₂₀, wherein N and R₂₀ are as above and M is 0 or an integer from 1-8.

R₁₀ may further preferably be selected from substituents forming an ester. For example, R₁₀ may be selected from compounds having the formula: -CO-R₂₀, wherein R₂₀ is as defined above.

R₁₀ may thus be also selected from acyls -CO-R₁₅ and -CO-R₁₇ as defined under (a) and (b) above.

More particularly, R₁₀ may be selected from compounds of formula (III) below: 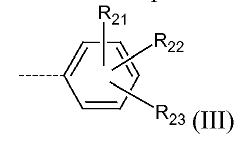 wherein the dotted line indicates the attachment of R₁₀ to the corresponding oxygen atom in formula (I). And wherein R₂₁, R₂₂ and R₂₃ are selected, independently of each other, from H, halogen (in particular Cl, Br and F), -CO-R₁₅, -CO-R₁₇ and C6-C12 aryl as defined below. Specific examples include -CO-Ph (Ph = phenyl), -CO-CH₂-Ph, -CO-Me (methyl), - CF₃, -Br, -Cl, -F. Any one of the substituents in (III) may be attached at carbon 2, 3 or 4 of the phenyl ring.

R₂₁, R₂₂ and R₂₃ may in particular be selected from substituents of formula (IV): 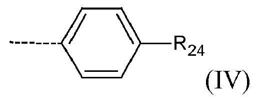 wherein the dotted line indicates the attachment of the substituent of formula (IV) to a ring carbon of the phenyl substituent R₁₀ of formula (III). And wherein R₂₄ is selected from H, C1-C5 alkyl as defined below, halogen, -CO-R₁₅, -CO-R₁₇, as defined above. For example, R₂₄ may be -CF₃, -CO-Ph, -CO-Me.

According to a preferred embodiment, R₁₀ is selected from α and β-furanosides and pyranosides, in particular of the following carbohydrates: D-glucose, D-mannose, D-galactose, N-acetyl-D-glucosamine, N-acetyl-D-galactosamine, L-fucose, L-rhamnose, L-daunosamine, D- and L-xylose, D- and L-lyxose, D- and L-arabinose, D- and L-ribose, D-and L-2-deoxyribose, D- and L-2-deoxyglucose, D- and L-2,6-dideoxyglucose, D- and L-2,6-dideoxygalactose, 2-deoxy-L-fucose, 2,3,6-trideoxy-L-threo-hexopyranose, 4-amino-2,3,6-trideoxy-L-arabino-hexopyranose, 4-O-methyl-L-arabino-hexopyranose, 3-amino-2,3,6-trideoxy-L-ribo-hexopyranose, 3-amino-2,3,6-trideoxy-3-C-methyl-L-lyxo-hexopyranose, 3-C-methyl-L-xylo-hexopyranose and 3-amino-2,3,6-trideoxy-3-C-methyl-L-arabino-hexopyranose.

The present invention provides compounds of formula (I), in which at least one hydrogen of the phenyl in formula (I) is substituted. In other words, at least one of the substituents R₄-R₈ is other than hydrogen. Such compound have surprisingly better anti-cancer characteristics than compounds in which there is no substituent of the phenyl. Without wishing to be bound by theory, it is speculated that the substituent in this position improves uptake of the compound by cancerous cells.

Accordingly, the at least one substituent of R₄-R₈ that is other than hydrogen may be selected, independently from the others, from halogens. For example, the phenyl of compound of formula (I) may be substituted with Br, F, or Cl, which may be situated at position 2, 3 or 4, preferably 4 (para) of the phenyl residue in formula (I).

Accordingly, the at least one substituent of R₄-R₈ that is other than hydrogen may be selected, independently from the others, from alkyls as defined below, preferably C1-C10 alkyls as defined below, which may be further substituted as indicated above. Examples are the alkyl examples already indicated above with respect to other substituents, such as R₃ and R₂₀ alkyl examples.

Furthermore, the at least one substituent of R₄-R₈ that is other than hydrogen may be selected, independently from the others, from alkenyls as defined below, preferably C1-C10 alkenyls as defined below, which may be further substituted as indicated above. Examples of alkenyls are indicated above with respect to R-R₁₁. A particularly preferred alkenyl is allyl.

The at least one substituent of R₄-R₈ that is other than hydrogen may also be selected, independently from the others, from alkenyls as defined below, preferably C1-C10 alkynyls as defined below, which may be further substituted as indicated below. Examples of alkenyls are indicated below.

Furthermore, the at least one substituent of R₄-R₈ that is other than hydrogen may be selected, independently from the others, from aryls as defined below. As indicated below, aryls include aromatic ring systems, with one (phenyl) or more rings. Aryls also include aralkyls (arylalkyls) as defined below, such as benzyl. These ring systems may be substituted as indicated above. Preferably, the aryl is further substituted as indicated above, for example with a halogen and/or with an alkyl. Preferably, one or more hydrogen atoms of said alkyl may be replaced by halogen. For example, the aryl is phenyl, substituted with trifluoromethyl. The one or more substituents of the aryl may be at any position of the aryl. If the aryl is phenyl, substituents of the aryl may be at 2, 3 or 4 position, but preferably at position 4.

Examples of compounds of the present invention when aryl is phenyl and a further substituted are provided in the examples. Also an example of an aryl being a heterocyclic compound (thiophene) is provided in the examples.

For the purpose of the present invention, the term "aryl" also encompasses alkylaryls (alkaryls, i.e. a mono-or polycyclic aromatic ring system substituted with an alkyl), and aralkyls, the latter including substituents that contain an aromatic ring or ring system of fused rings and which are attached to the phenyl group in formula (I) by way of an alkylene (also called "alkanediyl", for example, methylene, ethylene, propylene), alkenylene (also called: "alkenediyl", for example vinylene) or alkynylene, (also called "alkynediyl"). The alkylene, alkynylene or alkynylene is defined, it terms of carbon atoms, as the alkyls, alkenyl and alkynyls defined above. Preferably, the alkylene is selected from C1-C5, and the alkenylene and alkynylen from C2-C5. An examples of a substituent (R₄-R₈) in which a phenyl (aryl) is linked by a alkylene (methylene) to the phenyl in formula (I) is benzyl.

According to a preferred embodiment, at least one of R₄-R₈ is selected from a substituent according to formula (III) indicated above.

Furthermore, the at least one substituent of R₄-R₈ that is other than hydrogen may be selected, independently from the others, from acyls as defined below, preferably C1-C10 acyls as defined below, which may be further substituted as indicated above.

As indicated above, the alkyl, alkenyl, alkynyl, acyl or aryl of any one of R₄-R₈ may be further substituted as indicated above, and may comprise one or more heteroatoms. Heteroatoms may be selected as indicated above. According to preferred embodiment, heteroatoms are O.

According to a preferred embodiment, the alkyl, alkenyl, alkynyl, or aryl substituent is provided in the form of an ether, thus having the form -O-R₃₀, wherein R₃₀ may be selected from C1-C26 alkyl, C2-C26 alkenyl, C2-C26 alkynyl, C2-C26 acyl and C6-C26 aryl as defined below (optionally comprising one or more heteroatoms, linear, branched or cyclic, optionally further substituted as explained above. According to preferred examples, R₃₀ is selected from allyl (example for R₃₀ being an alkenyl), benzyl (example for R₃₀ being an aryl) or methyl (example for R₃₀ being an alkenyl). R₃₀ may in particular be an aromatic ring or fused ring system, such as a phenyl, benzyl, (arylalkyl, see below), wherein the aromatic ring may or not comprise heteroatoms, se below.

According to an embodiment, two or more of substituents R₄-R₈ are connected so as to form a ring fused to the phenyl in formula (I). The fused ring may comprise 3-8 carbons, may comprise heteroatoms and may be further substituted as indicated above.

According to a preferred embodiment, R₄-R₈ is selected from a substituted or unsubstituted aryl, said aryl being selected from a substituted or unsubstituted monocyclic aromatic ring (such as benzyl, thienyl and the like, see below) from an arylalkyl and from an arylalkoxyl, wherein the aryl in said arylalkyl and arylalkoxyl is a substituted or unsubstituted monocyclic aromatic ring. The monocyclic aromatic ring may be benzene or an aromatic heteroring as mentioned below. The arylalkyl (aralkyl) may be a benzyl or a 2-phenylethanyl, for example. The arylalkoxyl may have the following costitution: (aromatic ring-alkylene-O-), that is an alkoxyl in which one hydrogen is substituted with a mono- or polycyclic aromatic system, for example 5- or 6 membered aromatic ring.

The substituents R₄-R₈ which is other than hydrogen preferably comprises overall 1-26, 1-20, 1-15, 1-10, and most preferably 1-7 carbons, and, in addition, 0-10, 0-7, 0-5, 1-4, and most preferably 1-3 or 1-2 heteroatoms, as defined below.

In the compound of formula (I), all five, four, three, two or only one of the substituents R₄-R₈ may be different from hydrogen and be selected as indicated above. More preferably, only one selected from R₄-R₈ is different from hydrogen. Preferably, one substituent of R₅ and R₇ or substituent R₆ is different from hydrogen. Preferably, R₄, R₅, R₇, R₈ are hydrogen and R₆ is different from hydrogen (para position). Even more preferably, R₆ is different from hydrogen and comprises at least 1 carbon.

For the purpose of the present specification, any alkyl, acyl, alkenyl, and alkynyl may be linear, and, if the number of carbons is ≥3, they may be branched and/or cyclic.

For the purpose of the present specification, an aryl substituent encompasses any substituent comprising an aromatic moiety. Accordingly, the term aryl encompasses phenoxyl and benzyl (-CH₂-C₆H₅) groups, for example. An aryl, for the purpose of the present specification, also encompasses alkyls, alkenyls, alkynyls, as defined below, in which a hydrogen is substituted with phenyl or with another aromatic mono, bi-, tri-or polycyclic ring system.

Preferably, an alkyl is a C1-C15, more preferably a C1-C10 and most preferably a C1-C5 alkyl. If C ≥ 3, it may be linear, branched and/or cyclic. For example, the alkyl may be methyl, ethyl, propyl, butyl, pentyl, hexyl, heptyl and octyl. More specifically, alkyls may be selected from, *i*-propyl, *n*-propyl, *n*-butyl, *i*-butyl, *t*-butyl, *n*-pentyl, *n*-hexyl, *n*-heptyl, for example.

Preferably, an alkenyl is preferably, a C2-C15, more preferably a C2-C10 and most preferably a C2-C5 alkenyl. If C ≥ 3, it may be linear, branched or cyclic. For example, the alkenyl may be allyl, propenyl, butenyl, pentenyl, hexenyl. The alkenyl comprises at least one, but possibly two or more double bonds.

Preferably, an alkynyl is preferably, a C2-C15, more preferably a C2-C10 and most preferably a C2-C5 alkynyl. If C ≥ 3, it may be linear, branched or cyclic. For example, the alkynyl may be ethynyl, propynyl, butynyl, pentynyl, hexynyl. The alkynyl comprises at least one, but possibly two or more triple bonds. It may also comprise one or more double bonds.

An acyl substituent, for the purpose of the present specification, can also be regarded as alkyl or alkenyl substituent, for example, which is further substituted at C1 by a carbonyl group. An acyl is preferably, a C2-C15, more preferably a C2-C 10 and most preferably a C2-C5 acyl. The acyl may comprise one or more triple and/or double bonds. For example, the acyl may be acetyl, formyl, propionyl, benzoyl, acryl, sulfonyl from sulphonic or phosphonyl from phosphonic acids, for example.

The term aryl, for the purpose of the present invention, refers to an unsaturated aromatic carbocyclic group of from 6 to 26 carbon atoms having a single ring (e.g., phenyl) or multiple condensed rings (e.g., naphthyl or anthryl). Preferred aryls include phenyl, naphthyl and the like. Heterocyclic ring systems with aromatic properties, such as thiophene, pyridine, purine, furan, pyrrole, 1,3-oxazole, 1,3-thiazole, imidazole, indole, quinoleïne, isoquinoleïne, pyridazine, pyrazine, quinazoline, phthalazine, quinoxaline for example, are also considered as "aryls". For the purpose of the present specification, the term "aryl" includes alkylaryls, which are, for example, -alkylene-phenyl groups (see above), in which the alkylene has 1 - 10, preferably 1-3 carbons. Such aralkyl groups are exemplified by benzyl, phenethyl and the like. Preferably, the aryl is an C6-C15, more preferably C6-C12 aryl.

The alkyl, alkenyl, alkynyl, acyl and aryl may be substituted and may comprise one or more heteroatoms.

Heteroatoms are preferably selected from O, N, S, Se, P, B, halogen (F, Cl, Br, I). More preferably from O and N. Heteroatoms are not included in the number of carbon atoms of the compound indicated in the present specification. Residues R₁-R₁₀, and if applicable R₁₁, preferably comprise 1-10, 1-7, 1-5, 1-4, and most preferably 1-3 or 1-2 heteroatoms.

Substituents may be selected from:
- C1-C10, preferably C2-C5 alkyl, which may be linear, and, if ≥ C3, be cyclic or branched,
- C2-C15, preferably C3-C5 alkenyl, which may be linear, and, if ≥ C3, be cyclic or branched,
- halogen,
- =O, -OH, -NH₂;
- C6-C15, preferably C6-C10 aryl,
- C1-C15 acyl of formula -CO-R₁₄, wherein R₁₄ may be selected from H, C1-C₁₀ alkyl, C2-C25 alkenyl, C2-C15 aryl, C1-C15 acyl;
wherein these substituents may be further substituted and wherein, in particular, hydrogens may be replaced in part or totally by halogens;
- and combinations comprising two or more these;
- wherein, also in substituents, one or more hydrogen may be replaced by halogen.

The present invention also relates to pharmaceutical acceptable derivatives, in particular salts, of the compounds of the present invention.

Salts comprise, for example, salts with inorganic acids or organic acids like hydrochloric or hydrobromic acid, sulphuric acid, phosphoric acid, citric acid, formic acid, acetic acid, maleic acid, tartaric acid, benzoic acid, methanesulfonic acid, p-toluenesulfonic acid, and the like that are non toxic to living organisms or in case the compound (I) is acidic in nature with an inorganic base like an alkali or earth alkali base, e.g. sodium hydroxide, potassium hydroxide, calcium hydroxide and the like.

The compounds of the present invention may be used as pharmaceutically active principles. For example, they are useful as medicaments. The compounds of the present invention are preferably used as medicaments against cancer. Accordingly, the present invention relates to the use of the organometallic compounds comprising a structure of formula (I) in therapeutic cancer treatment. The compounds of the invention are useful for treating solid and non-solid neoplasms, such as human glioblastoma, but also for treating metastatic melanoma, breast cancer, prostate cancer, colorectal cancer, lymphoma, leukemia and myeloma, for example.

The invention provides compounds comprising a structure of formula (I), or prodrugs or solvates thereof ("active compounds"), for use in a method of treatment of the human or animal body. A method of treatment may comprise administering to such an individual a therapeutically-effective amount of the compound of the present invention, preferably in the form of a pharmaceutical composition. The term treatment as used herein in the context of treating a condition, pertains generally to treatment and therapy, whether of a human or animal (e.g. in veterinary applications), in which some therapeutic effect is achieved, for example, the inhibition of the progress of the condition, and includes a reduction in the rate of the progress, a halt in the rate of the progress, amelioration of the condition, and cure of the condition. The condition usually is associated with suffering, from psychological and/or physical pain, with the individual being in need of a treatment. Treatment as a prophylactic measure (i.e. prophylaxis) is also included.

The compound of the invention or pharmaceutical composition comprising the active compound may be administered to an individual by any convenient route of administration, whether systemically /peripherically or at the site of desired action, including but not limited to, oral (e.g. by ingestion), topical (including e.g. transdermal, intranasal, ocular, buccal and sublingual), pulmonary (e.g. by inhalation or insufflation therapy using an aerosol, e.g. through mouth or nose), rectal, vaginal, parenteral, for example by injection, including subcutaneous, intradermal, intramuscular, intravenous, intraarterial, intracardiac, intrathecal, subcuticula, subcapsular, intraorbital, intraperitoneal, intratracheal, subarachnoid, and intrasternal, by implant of a depot (e.g. subcutaneously or intramuscularly).

The compound of the present invention may be administered alone, but is preferably presented as a pharmaceutical composition (e.g. formulation) comprising at least one active compound together with at least one or more pharmaceutically carriers, buffers, as mentioned in WO 2006/018649, p. 16-20, "formulations", which reference is expressly incorporated herein by reference.

The present invention is described more concretely with reference to the following examples, which, however, are not intended to be understood as any kind of restriction of the scope of the present invention.

### Examples

### Example 1: Starting product: tert-butyl(3aR,4R,6aS)-4-formyl-2,2-dimethyltetrahydro-5H [1,3]dioxolo-[4,5-c]pyrrole-5-carboxylate (12)

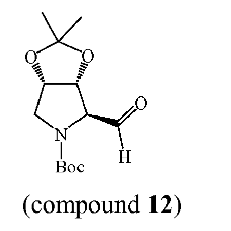

D-gulonolactone (25 g, 0.14 mol) was dissolved in acetone / DMP (5 : 1 vol : vol, 750 ml). *p-*toluenesulfonic acid was added until pH 3 and the solution was then stirred at 25°C until the starting material was consumed (control by TLC, AcOEt / petrol ether 4 : 1). The solution was neutralized with solid Na₂CO₃ and, after solvent evaporation *in vacuo,* the residue was poured into water and the aqueous phase was extracted with ethyl acetate (3 x 50 ml). The combined organic extracts were washed with brine (1 x 50 ml), dried with MgSO₄. After solvent evaporation *in vacuo,* 2,3:5,6-di-O-isopropylidene-D-gulono-1,4-lactone was obtained as a light yellow solid (24.536 g, 0.095 mol, 68 % yield). (In the Fleet's procedure (Tetrahedron 1988, 44, 2649-2655), once the solution has been neutralized with Na₂CO₃, the mixture was filtered through a Celite pad and then the product was recovered after solvent evaporation *in vacuo*).

The product obtained above (24.536 g, 0.095 mol) was added portionwise to a Red-Al solution in toluene / THF (49 ml of a 3.5 M solution in toluene diluted in 96 ml of anhydrous THF) at 0°C. The solution was stirred at 0°C for 5 h and then methanol was added until the excess of Red-Al was consumed. The solution was poured into a saturated solution of sodium potassium tartrate (150 ml) and the mixture was stirred for 2 h at 25°C. The organic phase was collected and the aqueous phase extracted with ethyl acetate (3 x 30 ml). The combined organic extracts were washed successively with a saturated solution of NaHCO₃ (1 x 30 ml) and brine (1 x 30 ml), then dried (MgSO₄). After solvent evaporation *in vacuo,* 5,6-Di-O-isopropylidene-D-gulitol was obtained as a white solid (18.689 g, 0.071 mol, 75% yield) without any further purification.

The subsequent reaction (esterification with methanesulfonyl chloride / pyridine) was performed following Fleet's procedure. The mesylate so obtained was not purified before its reaction with benzylamine forming N-benzyl-1,4-dideoxy-2,3:5,6-di-O-isopropylidene-1,4-imino-D-allitol in 40-55% yield, this also following Fleet's procedure.

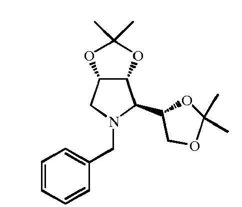

The excess of benzylamine was eliminated as its azeotrope with xylene and then the product was purified by flash chromatography (petroleum ether / diethyl ether 3 / 2).

N-benzyl-1,4-dideoxy-2,3:5,6-di-O-isopropylidene-1,4-imino-D-allitol was dissolved in acetic acid (80% vol:vol in water) and the solution was stirred at 60°C overnight. After evaporation of the solvent *in vacuo*, the product was purified by flash chromatography using pure ethyl acetate as eluent yielding 45-70% of N-benzyl-1,4-dideoxy-5,6-O-isopropylidene-1,4-imino-D-allitol.

This diol was then dissolved in methanol, Boc₂O (2 equivalent) was added and then Pd(OH)₂-C as catalyst under Argon atmosphere. The mixture was stirred under H₂ atmosphere for 3 h. The catalyst was filtered off on a Celite pad and the product was purified by flash chromatography (diethyl ether/ petroleum ether 4 : 1 to diethyl ether 100%) giving N-*tert-*butiloxycarbonyl-1,4-dideoxy-5,6-O-isopropylidene-1,4-imino-D-allitol in 82% yield.

The last step was the oxidation of the diol moiety of N-*tert*-butoxycarbonyl-1,4-dideoxy-5,6-O-isopropylidene-1,4-imino-D-allitol.

NaIO₄ (0.4083 g, 1.9 mmol, 2.7 eq) was added to a solution of the above product ( 0.202 g, 0.7 mmol) in methanol / water at 0°C and the solution was stirred for 1 h at 0°C. The solution was poured into water and ethyl acetate was added. The two phases were separated and the aqueous phase was extracted twice with ethyl acetate. The combined organic phases were washed with brine, dried with MgSO₄ and the solvent was evaporated *in vacuo* leading to pure *tert*-butyl(3*a*R,4R,6*a*S)-4-formyl-2,2-dimethyltetrahydro-5*H*-[1,3]dioxolo-[4,5-c]pyrrole -5-carboxylate (compound 12: 0.1844 g, 0.68 mmol, 97 % yield).

### Example 2: Reaction of protected 4-hydroxy-D-phenylglycine with triflic anhydride to generate the corresponding triflate methyl(2R)-[(tert-butoxycarbonyl)amino] {4{{(trifluoromethyl)sulfonyl}oxy}phenyl}acetate

4-hydroxy-D-phenylglycine (32) was commercially obtained. The carboxylic and amino groups were both protected in two successive steps, respectively, first by reflux in methanol solvent in the presence of SOCl₂, followed by Boc-protection: di-*tert*-butyl dicarbonate was added in H₂O/dioxane solvent in presence of triethylamine base at room temperature (r.t., 25°C). The protected 4-hydroxy-D-phenylglycine compound (34) was obtained with 83 mol.% yield.

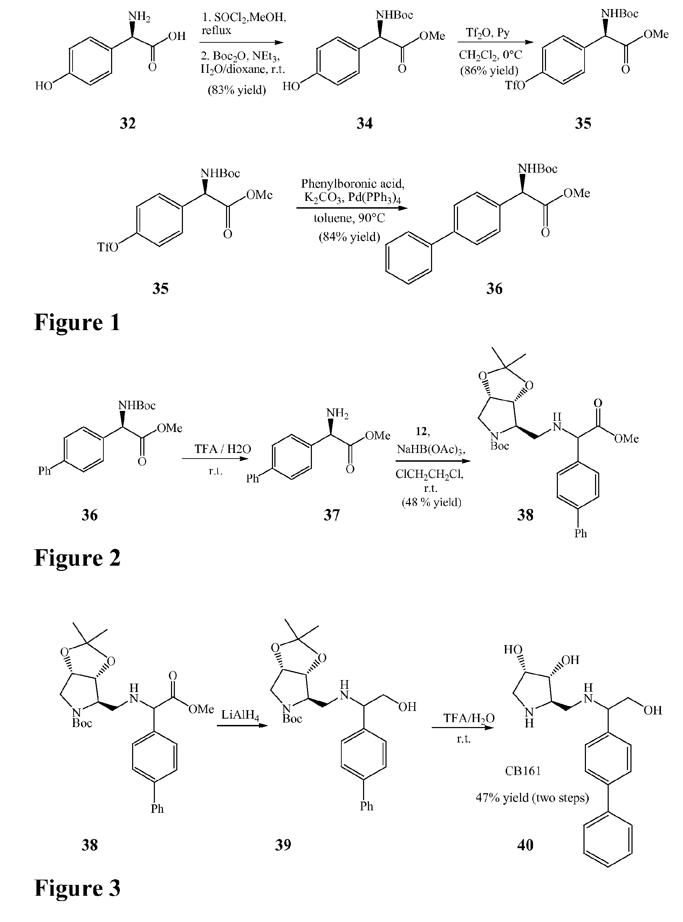

The protected intermediate reacted with triflic anhydride in dichlormethane solvent in presence of pyridine at 0°C to generate the corresponding triflate (35). The triflate Methyl(2R)-[(*tert*-butoxycarbonyl)amino]{4{{(trifluoromethyl)sulfonyl}oxy}phenyl}acetate was obtained in 84 mol.% yield (**Figure 1**).

### Example 3: Synthesis of Protected [1,1'-biphenyl]-D-glycine

The triflate compound (35) obtained in Example 2 was subjected to Suzuki cross-coupling with phenylboronic acid in toluene at 90°C using tetrakis(triphenylphosphine) palladium as catalyst and in presence of K₂CO₃ so as to obtain the desired biphenylglycine methyl ester (compound 36) in 84mol.% yield. Examples 2 and 3 are illustrated in **Figure 1****.**

### Example 4: Synthesis of Pyrrolidine derivative by Reductive Amination

The protection on the amino group of the biphenyl compound (36) obtained in Example 3 was removed in an aqueous solution of trifluoroacetic acid (TFA/H₂O = 4:1) at r.t. and purified following the same protocol described in Example 5 below with respect to removal of protective Boc and acetonide groups and purification. The amine (37) thus obtained was submitted to a reductive amination with the pyrrolidine-carbaldehyde shown in Example 1.

*Reductive Amination:* Accordingly, NaBH(OAc)₃ (0.1336 g, 0.63 mmol, 1.4 eq) was added portionwise, at r.t., to a stirred solution of 1,2-dichloroethane (4.5 ml) containing 0.1182 g (0.44 mmol) of the pyrrolidine-carbaldehyde shown in Example 1 (12) and 0.1024 g (0.42 mmol) of the deprotected amine (37) obtained above. After complete disappearance of reagents (reaction monitored by TLC), the solution was poured into a saturated aqueous solution of NaHCO₃ (5 ml per mmol). The organic phase was collected and the aqueous phase extracted with EtOAc (10 ml per mmol, 3 times). The combined organic extracts were washed with brine (10 ml per mmol, once) and dried (MgSO₄). Solvent evaporation *in vacuo* and flash chromatography (light petroleum/EtOAc 4:1) gave pure product (38), a colourless oil (0.0976 g, 0.20 mmol) at 48 mol.% yield.

The steps, starting materials and final product of Example 4 are shown in **Figure 2****.**

### Example 5: Synthesis of (2R,3R,4S)-2{{(1R)-1-[1,1'-biphenyl]-4-yl-2-hydroxyethyl]amino} methyl}pyrrolidine-3,4-diol (40)

The compound (38) obtained in Example 4 (*Tert*-butyl(3*a*R,4R,6*a*S)-4-{{[(1R)-1-biphenyl-4-yl-2-methoxy-2-oxoethyl]amino}methyl}-2,2-dimethyltetrahydro-5*H-*[1,3]dioxolo[4,5-c] pyrrole-5-carboxylate) was treated with LiAlH₄ to convert the ester group into the corresponding primary alcohol.

*Conversion of Ester into Primary Alcohol with LialH₄*: The ester (38) (0.0976 g, 0.20 mmol) was added portionwise to a cooled suspension (0°C) of LiAlH₄ (0.0167 g, 0.44 mmol) in anhydrous THF (5 ml). The solution was stirred at room temperature for 3 hours, then H₂O was added dropwise (0.5 ml) and the mixture was filtered on silica gel conditioned with EtOAc. After solvent evaporation *in vacuo*, the amino alcohol (39) (0.0772 g, 0.17 mmol) was obtained with 85% yield and deprotected as described below without further purification.

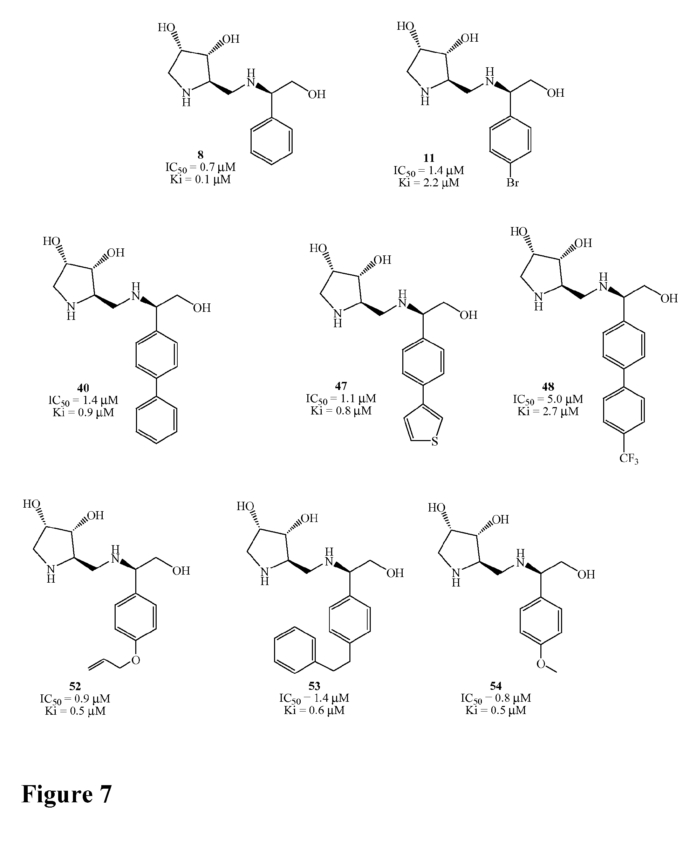

*Deprotection of the Boc and Acetonide Group*: 0.1 M solution of the amino alcohol (39) (0.17, 0.0772 g) in 1.7 ml CF₃COOH/H₂O (4:1 vol:vol) was stirred at room temperature for 2 hours. After solvent evaporation *in vacuo*, the residue was purified by flash chromatography on silica gel (CH₃CN/ NH₄OH 8:1). The pure product, compound (40), shown in Figure 7, (0.0271 g, 0.08 mmol, 47 mol.% yield) was recovered as a white solid. The steps, intermediate products and final product obtained are illustrated in Figure 3.

¹*H-NMR* (MeOH-d₄, 400MHz; bph = biphenyl): δ 2.61 (dd, ³J = 7.4, ²J = 12.1, 1H, CH₂-C(2)) 2.73 (dd, ³J = 5.0, ²J = 12.1, 1H, CH₂-C(2)) 2.88 (dd, ³J = 3.2, ²J = 12.2, 1H, H-C(5)) 3.12 (m, 1H, H-C(2)) 3.18 (dd, ³J = 5.1, ²J = 12.2, 1H, H'-C(5)) 3.61 (dd, ³J = 8.7, ³J = 10.7, 1H C*H*CH₂OH) 3.69 (m, 2H, CHC*H*₂OH) 3.82 (dd, ³J = 4.4, ³J = 8.3, 1H, H-C(3)) 4.05 (m, 1H, H-C(4)) 7.32 (m, 1H, H-C(4') (bph)) 7.43 (m, 4H, arom. H (bph)) 7.58 (m, 4H, arom. H (bph)).

### Example 6: Synthesis of (2R,3R,4S)-2{{{(1R)-2-hydroxy-1-[4-(3-thienyl)phenyl]ethyl amino}methyl}pyrrolidine-3,4-diol (47)

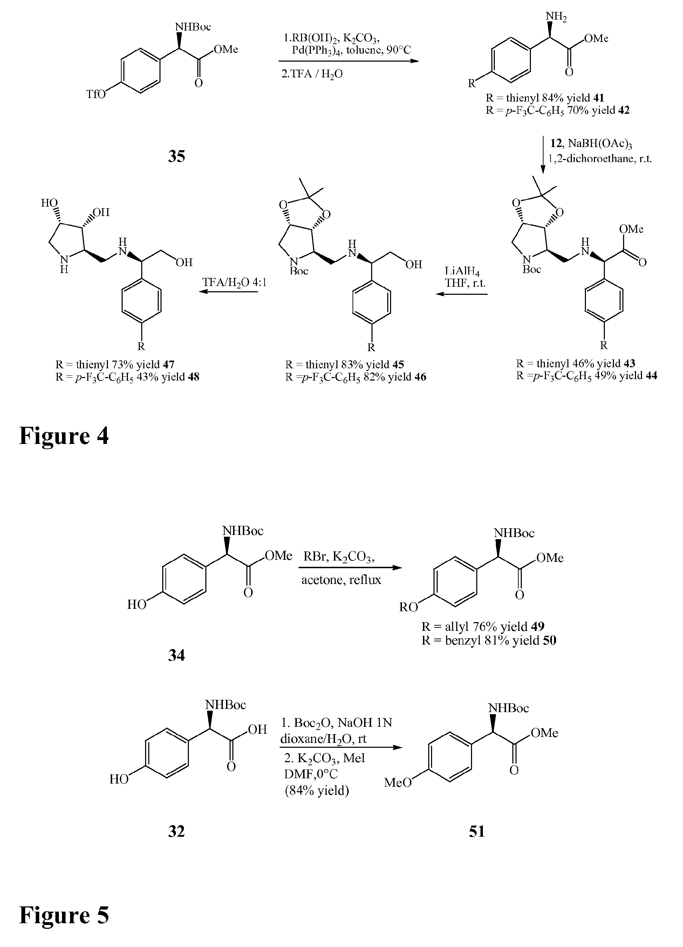

The steps for obtaining compound 47 and 48 (Example 7) are illustrated in **Figure 4****.** Starting from the triflate compound (35) obtained in Example 2, Suzuki cross-coupling was performed in analogy with Example 3, however using commercially obtained 3-thiopheneboronic acid instead of phenylboronic acid in the coupling reaction, followed by deprotection of the amino group (TFA in H₂O, r.t., Examples 4 and 5) so as to obtain the corresponding thienyl compound (41) in 84% yield.

The reductive amination was performed as described in Example 4, by adding 1.4 equivalents NaBH(OAc)₃ and using thienyl compound (41) as amine. Flash chromatography with light petroleum/EtOAc 3:1 gave product (43), a colourless oil with 46% yield.

As described in Example 5, compound (43) was converted into a primary alcohol (83% yield) when treated with LiAlH₄, followed by deprotection and purification. The pure product (compound **47**, shown in Figure 7) was recovered as a white foam with 73 mol.% yield.
*¹H-NMR* (MeOH-d, 400MHz; thi = thienyl): δ 2.46 (dd, ³J = 7.8, ²J = 12.6, 1H, CH₂-C(2)) 2.60 (m, 1H, CH₂-C(2)) 2.80 (d, ²J = 10.8, 1H, H-C(5)) 3.07(m, 2H, H-C(2) and H'-C(5)) 3.58 (m, 3H, C*H*CH₂OH and CHC*H*₂OH ) 3.71 (m, 1H H-C(3)) 3.99 (m, 1H, H-C(4)) 7.27 (d, ³J =8.0, 2 arom. H (Ar)) 7.38 (m, 2H, arom. H (thi)) 7.54 (m, 1H, arom. H (thi)) 7.57 (d, ³J = 8.1, 2H, arom. H (Ar)).

### Example 7: Synthesis of (2R,3R,4S)-2-{{{(1R)-2-hydroxy-1-[4'-(trifluoromethyl)-[1,1'-biphenyl]-4-1]ethyl}amino} methyl}pyrrolidine-3,4-diol (48)

Starting from the triflate compound (35) obtained in Example 2, Suzuki cross-coupling was performed in analogy with Example 3, however using commercially obtained 4-(trifluoromethyl)phenylboronic acid instead of phenylboronic acid in the coupling reaction, followed by deprotection of the amino group (TFA in H₂O, r.t., Examples 4 and 5) so as to obtain the corresponding p-F₃C-C₆H₅ compound (42) in 70.4% yield.

The reductive amination was performed as described in Example 4, by adding 1.4 equivalents NaBH(OAc)₃ and using the p-F₃-C₆H₅-compound (42) as amine. Flash chromatography with light petroleum/EtOAc 3:1 gave product (44), a light yellow oil with 49% yield.

As described in Example 5, compound (43) was converted into a primary alcohol (82% yield) when treated with LiAlH₄, followed by deprotection and purification. The pure product (48, shown in Figure 7, was recovered as a white foam with 43 mol.% yield.

*¹H-NMR* (MeOH-d₄, 400MHz): δ 2.81 (dd, ³J = 8.8, ²J = 11.7 1H, CH₂-C(2)) 2.94 (bd, ²J = 11.7, 1H, CH₂-C(2)) 3.27 (m, 1H, H-C(5)) 3.43 (m, 2H, H-C(2) and H'-C(5)) 3.67 (m, 2H, C*H*CH₂OH and 1H CHC*H*₂OH) 3.88 (dd, ³J = 4.8, ²J = 7.6, 1H C*H*CH₂OH) 3.93 (dd, ³J = 4.0, ³J = 8.0, 1H, H-C(3)) 4.20 (m, 1H, H-C(4)) 7.49 (m, 2 arom. H) 7.69 (m, 4 arom. H) 7.77 (m, 2 arom. H).

### Example 8: Synthesis of Allyl Ethers of Protected 4-hydroxy-D-phenylglycine (32 and 34)

The carboxylic and amino groups of commercially obtained 4-hydroxy-D-phenylglycine (32) are protected to obtain compound (34) as indicated in Example 2.

The protected 4-hydroxy-D-phenylglycine (34) was refluxed in acetone containing allyl bromide in presence of K₂CO₃, so as to obtain allyl derivatives (49) in 76% yield, as is shown in **Figure 5****.**

### Example 9: Synthesis of (2R,3R,4S)-2{{{(1R)-2-hydroxy-1-[4-(prop-2-enyloxy)phenyl] ethyl}amino}methyl} pyrrolidine-3,4-diol (52)

The amino group of the allyl ether of protected 4-hydroxy-D-phenylglycine (49) was deprotected and purified as described in Examples 4 and 5. The amine thus obtained was used in reductive amination.

The reductive amination was performed as described in Example 4, by adding 1.4 equivalents NaBH(OAc)₃. Flash chromatography with light petroleum/EtOAc 2:1 gave a light yellow oil with 43% yield.

As described in Example 5, the purified compound obtained in the preceding step was converted into a primary alcohol (83% yield) when treated with LiAlH₄, followed by deprotection and purification. The pure product (52), shown in Figure 7, was recovered as a white solid with 44 mol.% yield.

*¹H-NMR* (MeOH-d₄, 400MHz; all = allyl): δ 2.52 (dd, ³J = 7.6, ²J = 12.0, 1H, CH₂-C(2)) 2.63 (dd, ³J = 5.2, ²J = 12.0, 1H, CH₂-C(2)) 2.81 (dd, ³J = 3.4, ²J = 12.3, 1H, H-C(5)) 3.03 (m, 1H, H-C(2)) 3.12 (dd, ³J = 5.2, ²J = 12.3, 1H, H'-C(5)) 3.58 (m, 3H C*H*CH₂OH and CHC*H*₂OH) 3.69 (dd, ³J = 4.4, ³J = 8.4, 1H, H-C(3)) 4.01 (m, 1H, H-C(4)) 4.52 (d, ³J = 5.12, 2H, CH₂=CHC*H*₂O) 5.22 (d, ²J = 10.0, 1H, H_{cis}-C(3); all (cis rel. to H-C(2); all)) 5.38 (d, ²J = 17.8, 1H, Hₜᵣₐₙₛ-C(3); all (trans rel. to H-C(2); all)) 6.04 (m, 1H, CH₂=C*H*CH₂O) 6.89 (d, ³J = 8.6, 2H, Ar-H) 7.25 (d, ³J = 8.6, 2H, Ar-H).

### Example 10: Synthesis of (2R,3R,4S)-2{{(1R)-2-hydroxy-1-{4-[(phenylmethoxy)phenyl] hydroxyethyl}amino} methyl}pyrrolidine-3,4-diol (53)

Compound (53) was synthesized according to the same procedure as given in Examples 8 and 9. Instead of allyl bromide in Example 8, benzyl bromide was used, and the benzyl derivative (50) was obtained in 81% yield, as shown in **Figure 5**. Deprotection of the amino group of (50), and reductive amination with 1.4 equivalent NaBH(OAc)₃ were done as described in Example 4. Following flash chromatography as in Example 9, the product obtained was a colourless oil (41% yield).

As described in Example 5, the purified compound obtained in the preceding step was converted into a primary alcohol (83% yield) when treated with LiAlH₄, followed by deprotection and purification by flash chromatography. In this Example, contrary to Example 5, CH₃CN/ NH₄OH (6:1) was used for flash chromatography. The pure product (**53**), shown in **Figure 7**, was recovered as a pale yellow foam with 42% yield.

*¹H-NMR* (MeOH-d, 400MHz): δ 2.61 (dd, ³J = 7.9, ²J = 12.3, 1H, C*H*₂-C(2)) 2.74 (dd, ³J = 4.7, ²J = 12.3, 1H, C*H*₂-C(2)) 2.96 (d, ²J = 12.2, 1H, H-C(5)) 3.18 (m, 1H, H-C(2)) 3.24 (dd, ³J = 4.5, ²J = 12.2, 1H, H'-C(5)) 3.56 (dd, ³J = 8.8, ²J = 10.8, 1H CHC*H*₂OH) 3.62 (dd, ³J = 4.4, ²J = 10.8, 1H, CHC*H*₂OH) 3.73 (m, 2H, C*H*CH₂OH and H-C(3)) 4.10 (m, 1H, H-C(4)) 5.09 (s, 2H, PhC*H*₂) 7.00 (d, ³J = 8.5, 2 arom. H (Ar)) 7.29 (d, ³J = 8.5, 2 arom. H (Ar)) 7.32 (m, 1 arom. H (Ph)) 7.38 (m, 2 arom. H (Ph)) 7.44 (m, 2 arom. H (Ph)).

### Example 11: Synthesis of (2R,3R,4S)-2{{[(1R)-2-hydoxy-1-(4-methoxyphenyl)ethyl] amino}methyl}pyrrolidine-3,4-diol (54)

Methyl ether (51) was prepared starting from unprotected 4-hydroxy-D-phenylglycine (32), in which the amino group was protected first according to the procedure described in Example 2 (Boc₂O, NaOH 1N, dioxane/H₂O, r.t.). Then, both carboxylic and phenol groups were treated with Mel at 0°C in DMF and in presence of K₂CO₃, leading to the desired 4-methoxy-N-Boc-D-phenylglycine methyl ester (51) (**Figure 5**).

Then, the same procedure described in Example 9 was repeated: The amino group of the methoxy methyl ester (51) was deprotected and the resulting amine was purified as described in Examples 4 and 5. The amine thus obtained was used in reductive amination.

The reductive amination was performed as described in Example 4, by adding 1.4 equivalents NaBH(OAc)₃. Flash chromatography with light petroleum/EtOAc 2:1 gave a yellow oil with 52% yield.

As described in Example 5, the purified compound obtained in the preceding step was converted into a primary alcohol (83% yield) when treated with LiAlH₄, followed by deprotection and purification. In this Example, contrary to Example 5, CH₃CN/ NH₄OH (6:1) was used for flash chromatography. The pure product (54), shown in **Figure 7**, was recovered as a brown solid with 76 mol.% yield.

*¹H-NMR* (MeOH-d₄, 400MHz): δ 2.53 (dd, ³J = 7.8, ²J = 11.8, 1H, CH₂-C(2)) 2.66 (dd, ³J = 4.6, ²J = 11.8, 1H, CH₂-C(2)) 2.84 (dd, ³J = 3.2, ²J = 12.2, 1H, H-C(5)) 3.07 (m, 1H, H-C(2)) 3.14 (dd, ³J = 5.2, ²J = 12.0, 1H, H'-C(5)) 3.62 (m, 3H, C*H*CH₂OH and 2H CHC*H*₂OH) 3.75 (dd, ³J = 4.6, ³J=9.0, 1H, H-C(3)) 3.80 (s, 3H, *H*₃CO) 4.05 (m, 1H, H-C(4)) 6.93 (d, ³J = 8.8, 2 arom. H) 7.29 (d, ³J = 8.8, 2 arom. H).

### Example 12: Synthesis of (2R,3R,4S)-2{{[(1R)-1-(4-Bromophenyl)-2-hydroxyethyl]amino} methyl}pyrrolidine-3,4-diol (11)

### Synthesis of 2-(R)-(4-bromophenyl)glycinol intermediate (13)

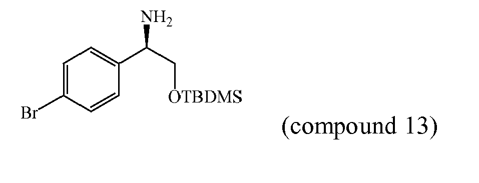

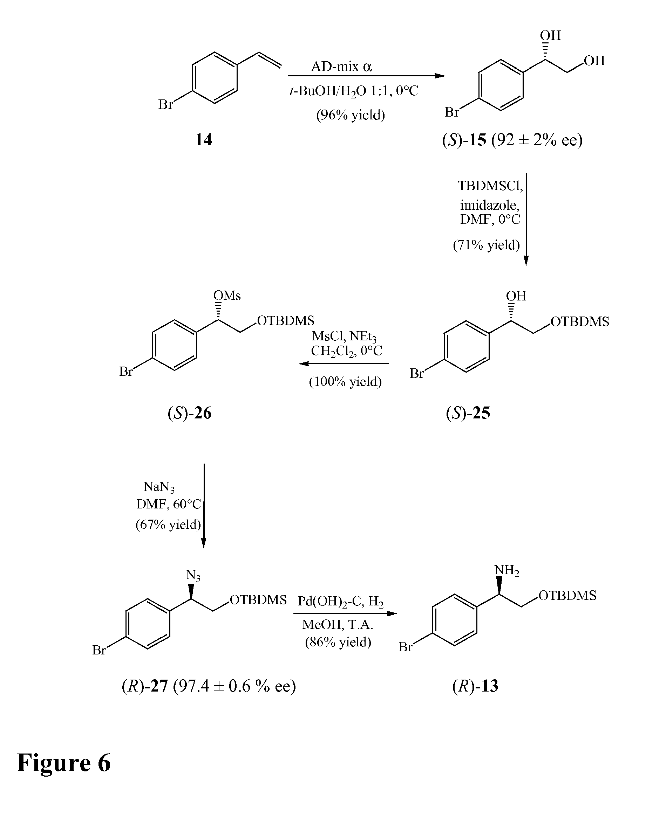

Enaniomerically pure compound 13 was synthesised by Sharpless asymmetric dihydroxylation (E. Jacobsen, et al. J. Am. Chem. Soc.1988, 110, 1968-1970). Sharpless asymmetric dihydroxylation of 4-bromostyrene, in presence of AD-mix α, produced the corresponding diol (*S*)-**15** in 96% yield and 92 % enantiomeric excess. Selective protection of the primary alcohol as a (*tert*-butyl)dimethylsilyl ether (TBDMS) followed by mesylation of the secondary alcohol and displacement through nucleophilic substitution of the mesylate with sodium azide, led to the expected azido alcohol **27**. Finally, palladium catalyzed hydrogenation reduced (*R*)-**27** into (*R*)-**13** with good yield and enantiomeric excess (**Figure 6**). The absolute configuration and the enantiomeric excess for both diol (*S*)-**15** and the azide (R)-27 were evaluated by NMR applying Mosher's method. The ¹H-NMR spectra of the two MTPA esters of the monoprotected diol (*S*)-**25** allow the assignment of the (*S*) configuration to diol **15**. The enantiomeric excess (92 ± 2%) was determined from the ¹⁹F-NMR spectra of the two MTPA diastereoisomers. Assignment of the absolute configuration and calculation of the enantiomeric excess (97.4 ± 0.6 %) of protected amino alcohol **13** were made on the basis of the ¹H-NMR data of the diastereoisomeric MTPA-amides of **13**.

Reductive amination of compound (12) (Example 1) with the semiprotected phenylglycinol (13) was performed as described in Example 4, by adding 1.4 equivalents NaBH(OAc)₃. Flash chromatography with light petroleum/EtOAc 4:1 afforded compound (28) (below) in 51 % yield.

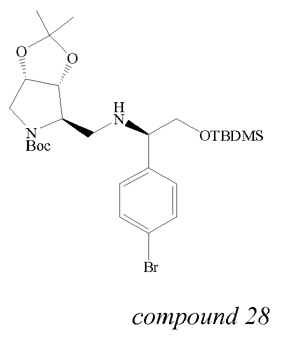

Deprotection with TFA / H₂O (4:1 vol : vol) at r.t. and purification by flash chromatography (CF₃COOH /NH₄OH 5:1) afforded compound (11), which is shown in **Figure 7****.**

*¹H-NMR* (MeOH-d₄, 400MHz): δ 2.74 (dd, ³J = 8.8, ²J = 13.3, 1H, CH₂-C(2)) 2.89 (dd, ³J = 4.4, ²J = 13.3, 1H, CH₂-C(2)) 3.22 (dd, ³J = 2.1, ²J = 12.5, 1H, H-C(5)) 3.43 (m, 2H, H'-C(5) and CH(2)) 3.60 (dd, ³J = 8.3, ²J = 11.0, 1H CHC*H*₂OH) 3.66 (dd, ³J = 4.5, ²J = 11.0, 1H, CHC*H*₂OH) 3.79 (dd, ³J = 4.5, ³J = 8.3, 1H, C*H*CH₂OH) 3.94 (dd, ³J = 4.1, ³J = 7.9 , 1H, H-C(3)) 4.22 ( m,1H, H-C(4)) 7.32 (m, 4H, arom. H).

### Example 13: Determination of Cell Growth Inhibition by dihydroxypirolidine derivatives

### Materials and methods

Cell lines and reagents. The tumor cell lines used for the in vitro evaluation of cell growth inhibition by dihydroxypirolidine derivatives were U87 (glioblastoma), PC3 (prostate cancer), A549 (lung carcinoma), MDA-MB231, MCF7, BT474, and SKBR3 (breast cancer). Cells were grown in 10-cm culture dishes with McCoy medium containing 10% fetal calf serum (FCS) and antibiotics at 37°C and 5% CO₂. The dihydroxypirolidine derivatives CB161, CB181, and CB183 were weighted and dissolved in DMSO to prepare a stock solution concentrated 100 mM.

Viability assays. For the viability assays, 5 x 10⁴ cells/well were plated in 200 µl medium in 96 well plates. 48 hours later, the dihydroxypirolidine derivatives were added to the wells at concentrations ranging between 10⁻² and 400 µM, such that the vehicle DMSO never exceeded 0.4%. Each drug concentration was tested in duplicate. Viability was determined 72 hours later using CellTiter 96 Aqueous1 (Promega) according to the manufacturer's instructions. Incubation times with CellTiter96 Aqueous1 ranged between 2 and 4 hours. Plates were read with a spectrophotometer (Labsystems iEMS Reader MF) at 490 nm wave length. IC50s were estimated using GraphPad Prism4.

Microscopy. Cells were imaged using the 40X magnification of a Zeiss AXIOVERT200 microscope, camera Qlympus C-4040ZOOM. The image files were downloaded using the software Olympus CAMEDIA Master 2.5.

### Results

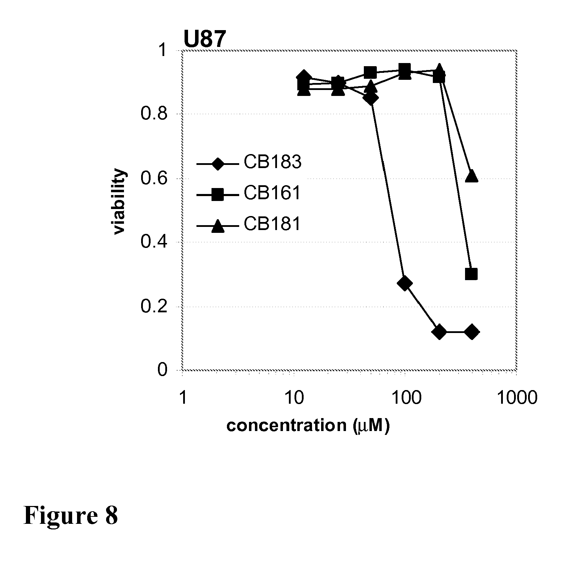

The three dihydroxypirolidine derivatives CB161, CB181, and CB183 were evaluated for their capacity to inhibit cell growth on seven established human tumor cell lines of different histology (glioblastoma, prostate cancer, lung cancer, and breast cancer). CB183 showed potent cytotoxic activity in all the cell lines tested for concentrations <200 µM (Table 1). CB161 and, to a lesser extent, CB181 also exhibited growth inhibition effects although with IC50s >200 µM (**Figure 8**).

**Table 1: Cytotoxicity of a compound of the present invention on different cancer cell lines**

| **Cell lines** | **CB183** |
|---|---|
| | IC50 (µM) |
| A549 | 152 |
| PC3 | 132 |
| U87 | 80 |
| MCF7 | 76.7 |
| MDA-MB-231 | 65.8 |
| BT474 | 93.3 |
| SKBR3 | 48.3 |

## Claims

1. A compound of formula (I): 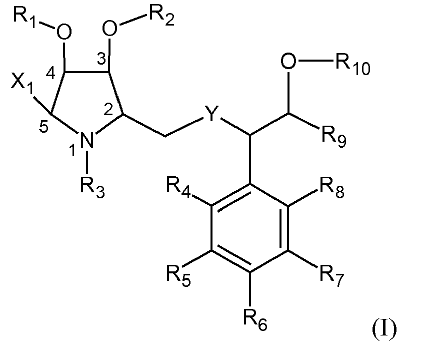 wherein:
X₁ is selected from H, -CH₂-O-R, or -O-R;
Y is selected from -NR₁₁-, -O-, CO, CH(OH), CH(NH₂), -CH₂-, -S-, -SO-, -SO₂-;
R, R₁, R₂, R₃, R₄, R₅, R₆, R₇, R₈ R₉, R₁₀ and, if applicable R₁₁, independently of each other, from H, C1-C26 alkyl, C1-C26 acyl, C2-C26 alkenyl, C2-C26 alkynyl, C6-C26 aryl,
wherein said alkyl, acyl, alkenyl, alkynyl, and aryl, may be further substituted and may comprise one or more heteroatoms;
wherein, furthermore, R₁, R₂ may form an acetal of formula (II): 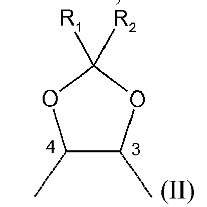 wherein, in said acetal, R₁ and R₂ are as defined above;
wherein at least one of R₄-R₈ is different from hydrogen;
wherein any carbon-bound H in any of the residues R₁-R₁₁ may be replaced by halogen;
wherein -R₉ may also represent a carbonyl group (=O);
wherein the compound of formula (I) may be provided in the form of a pharmaceutically acceptable salt.

2. The compound of claim 1, wherein R₄-R₈ is selected from halogen, aryl, alkyl, alkoxyl, alkenoxyl, wherein said aryl, alkyl, alkyoxyl, and alkenoxy may be further substituted.

3. The compound of claim 1 or 2, wherein R₄-R₈ is selected from a substituted or unsubstituted aryl, said aryl being selected from a substituted or unsubstituted monocyclic aromatic ring, from an arylalkyl and from an arylalkoxyl, wherein the aryl in said arylalkyl and arylalkoxyl is a substituted or unsubstituted monocyclic aromatic ring.

4. The compound of any one of the preceeding claims, wherein the aryl is selected from phenyl, alkylphenyl, phenylalkyl (e.g. benzyl), phenylalkoxyl, wherein in any alkyl one, more or all hydrogen atoms may be substituted by halogen.

5. The compound of claim 1, wherein the aryl of R₄-R₈ is selected from an optionally substituted C6-C25 aroxyl (e.g. phenoxyl), aracyl (e.g. benzoyl), C7-C25 aralkyl (including benzyl), and a mono- bi or tricyclic aromatic system (including phenyl), which may optionally be substituted by a C1-C10 alkyl, wherein one or more hydrogens of said aroxyl, aracyl, aralkyl, said mono- bi or tricyclic aromatic aromatic system and of said C1-C10 alkyl are optionally substituted by one or more halogens.

6. The compound of Claim 1, wherein at least on of the substituents R₄-R₈ is selected from halogen, a five- or six-membered heterocycle, a C1-C26 alkyl, a C2-C26 alkenyl, a C2-C26 alkynyl, C1-C26 acyl, C6-C26 aryl, and -O-R₁₃; wherein R₁₃ represents C1-C26 alkyl, C2-C26 alkenyl, C2-C26 alkynyl, C6-C26 aryl (including alkyls substituted with aryls), C1-C26 acyl; and, wherein any of these alkyls, alkenyls, alkynyl, aryls may be further substituted and wherein one or more hydrogens may be substituted by halogen.

7. The compound of any one of claim 1, wherein at least one of R₄-R₈ is selected from an optionally substituted C1-C10 alkyl, phenyl and benzyl, wherein said optional substitutes are selected from halogen and C1-C15 alkyl in which one or more hydrogens are optionally substituted by halogen.

8. The compound of any one of the preceding claims, wherein R₁ and R₂ are selected, independently of each other, from H and C1-C10 alkyl, C1-C10 acyl, and C6-C18 aryl,
wherein said alkyl, acyl and aryl may be substituted by -OH, -C=O, and C6-C18 aryl, and
wherein any one or all hydrogen atoms in any of said alkyl, acyl and aryl may be replaced by a halogen.

9. The compound of claim 8, wherein said C6-C18 aryl is selected from a C6-C18 aracyl and a C6-C18 mono- bi and tricylcic aromatic system (including phenyl).

10. The compound of any one of the preceding claims, wherein R₃ and if applicable, R₁₁, are selected, independently of each other, from H, alkyl, acyl, aryl, benzoyl, each of which may optionally be substituted.

11. The compound of any one of the preceding claims, wherein R₁₀ is selected from:
H, C1-C15 alkyl, C2-15 alkenyl, C1-15 alkoxyl, C6-15 aryl, α and β furanosides and pyranosides;
wherein said alkyl, alkenyl, alkoxyl, aryl optionally comprises one or more oxygen heteroatoms, and wherein one or more hydrogen may be replaced by halogen.

12. The compound of any one of the preceding claims, wherein R₁₀ is selected from α and β-furanosides and pyranosides of the following carbohydrates: D-glucose, D-mannose, D-galactose, N-acetyl-D-glucosamine, N-acetyl-D-galactosamine, L-fucose, L-rhamnose, L-daunosamine, D- and L-xylose, D- and L-lyxose, D- and L-arabinose, D- and L-ribose, D-and L-2-deoxyribose, D- and L-2-deoxyglucose, D- and L-2,6-dideoxyglucose, D- and L-2,6-dideoxygalactose, 2-deoxy-L-fucose, 2,3,6-trideoxy-L-threo-hexopyranose, 4-amino-2,3,6-trideoxy-L-arabino-hexopyranose, 4-O-methyl-L-arabino-hexopyranose, 3-amino-2,3,6-trideoxy-L-ribo-hexopyranose, 3-amino-2,3,6-trideoxy-3-C-methyl-L-lyxo-hexopyranose, 3-C-methyl-L-xylo-hexopyranose and 3-amino-2,3,6-trideoxy-3-C-methyl-L-arabino-hexopyranose.

13. The compound of any one of the preceding claims, wherein one or more of R₁, R₂, R₃, if applicable: R₈, R₁₀ and X are hydrogen.

14. The compound according to any one of the preceding claims, wherein any alkyl, in particular the alkyls of the C1-C26 alkyl of R₁ and/or R₂, the C1-C15 alkyl of R₉, the C1-C26 alkyl of R₁₀, the C1-C10 alkyl substituent referred to in claim 2, the C1-C10 alkyl substituent referred to in claim 3, the alkyl of R3, the alkyl of R11 are linear alkyls.

15. The compound of any one of the preceding claims, having the structure of formula (V). 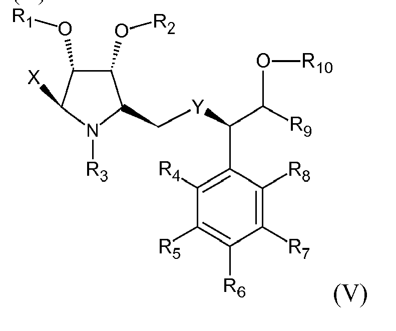 wherein R₁-R₁₀ are as defined above.

16. The compound of any one of the preceding claims, having the structure of formula (III): 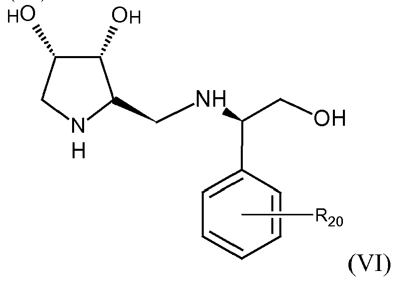 wherein R₂₀ defined as R₄-R₈ above, with the proviso that R₂₀ is not hydrogen.

17. The compound of any one of the preceding claims for use as a medicament.

18. The compound of any one of the preceding claim in the treatment of cancer.

19. The compound of any one of the preceding claims in the treatment of a non-solid neoplasm.

20. A method for producing a compound according to formula (I), in which
Y is -N-R₁₁;
X, R₃, R₁₁ and X are H,
R₄-R₈ are as indicated in claim 1;
-R₉ represents a carbonyl group (=O);
wherein R₁, R₂ and R₁₀ may be as indicated in claim 1, in particular they may be H or suitable protective groups;
wherein said method comprises the steps of:
a) providing a compound of formula (X): 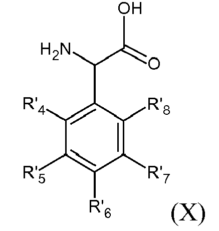 in which at least one of R'₄-R'₈ is selected from -OH, or halogen, the other being hydrogen;
b) protecting the free amino and hydroxyl groups visible in compound (X) (other than R'₄-R'₈);
c) if the at least one of R'₄-R'₈ is -OH, reacting compound (X) with a triflate, so generate the corresponding triflate;
d) conducting a Suzuki reaction, in which the halogen, or, if applicable, the triflate added in the preceding step, is replaced by any residue R₄-R₈ other than hydrogen as defined in claim 1, so as to obtain compound (XI); 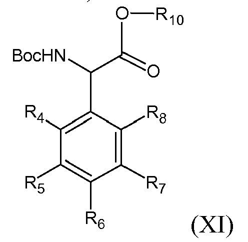 wherein R₄ and R₈ are as defined above.
e) deprotecting the amino group of the compound (XI) obtained in the preceding step; and,
f) conducting a reductive amination of a compound of formula (XII) with said deprotected compound (XI) of the preceding step comprising a free amino group; 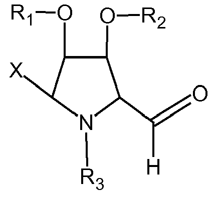 wherein X, R₁, R₂ and R₃ are as defined above, with the proviso that R₁, R₂ and R₃ are different from H and that each of X, R₁, R₂ and R₃ is free of any free group intervening in the reductive amination of this step;

21. The method of claim 20, wherein, in the Suzuki reaction in step d) is conducted with a boron ester or boronic acid carrying, as an organic residue, a residue -R₂₁defined as R₄-R₈, so that -R₂₁ replaces the triflate or halogen group of compound (X).

22. The method of claim 20 or 21, further comprising the step of:
g) conducting a reduction so that R₉ is different from =O.

23. The method of any one of claims 20-22, wherein step f) combining the protected fragment in step b) with the compound of formula (XII) is conducted before steps c) and d), so that the Suzuki reaction replacing any residue of R'₄-R'₈ with R₄-R₈ is conducted following the reductive amination of step f).

24. The method of step 23, wherein in step b), the free amino group is not protected but one, or, if applicable, two hydroxy groups in compound (X) are protected before conducting reductive amination.
